# NEUE EUROPÄISCHE PATENTSCHRIFT

(11) **EP 0 462 526 B2**
(45) Veröffentlichungstag und Bekanntmachung des Hinweises auf die Entscheidung über den Einspruch: **22.12.1999**
(45) Hinweis auf die Patenterteilung: 12.02.1997
(21) Anmeldenummer: 91109824.2
(22) Anmeldetag: 15.06.1991
(51) Int. Cl.: B60S 1/38, B60S 1/48

(54) **Verfahren zur Reinigung von Glasscheiben**
Procedure for the cleaning of glass panes
Procédé de nettoyage pour le nettoyage des vitres

(30) Priorität: 20.06.1990 DE 4019588
(43) Veröffentlichungstag der Anmeldung: 27.12.1991
(73) Patentinhaber: ROBERT BOSCH GMBH, 70442 Stuttgart (DE)
(72) Erfinder: Brill, Klaus, Dr. Dipl.-Physiker, W-7015 Korntal-Muenchingen (DE); Rach, Elmar, Dr. Dipl.-Physiker, W-7247 Ditzingen-Heimerdingen (DE)

(56) Entgegenhaltungen:
- WO-A-91/11349
- DD-A- 241 083
- DD-A- 275 878
- DE-A- 3 803 491
- GB-A- 1 366 910
- GB-A- 2 004 458
- GB-A- 2 224 743
- US-A- 1 919 236
- US-A- 3 012 384
- US-A- 4 016 623
- US-A- 4 045 838
- PATENT ABSTRACTS OF JAPAN vol. 012, no. 490 (M-779)21. Dezember 1988; & JP-A-63 212 474 (TAKAYUKI NAKAJIMA) 5. September 1988
- PATENT ABSTRACTS OF JAPAN vol. 015, no. 203 (M-1116)24. Mai 1991; & JP-A-03 054 051 (NITTO KAGAKU KK) 8. März 1991
- Marke SIDOL Anmeldenummern S10328 und S18989 (Warenzeichenblätter III,17, Seite 2/44 v. 15.09.1952 und II, S. 82 v. 14.01.1967)

## Beschreibung

### Stand der Technik

Die Erfindung betrifft ein Verfahren zur Reinigung von Kraftfahrzeugfront- und -heckscheiben, die von einem Scheibenwischer überstrichen werden, bei dem Wasser und ein Reinigungsmittel auf die Scheibe aufgebracht werden.

Bei der Bewegung eines Scheibenwischers über eine nasse Glasscheibe bleibt hinter dem Wischgummi bzw. der Wischlippe ein dünner Wasserfilm zurück. In der Praxis, insbesondere im Fahrbetrieb von Kraftfahrzeugen, werden auch gut gereinigte und dadurch hydrophile Scheibenoberflächen schon nach kurzer Zeit durch Schmutzablagerungen hydrophob. Es genügen bereits Schichten von wenigen Moleküllagen, um die Oberflächeneigenschaften des Glases völlig zu verändern. Bei den hydrophobierenden Substanzen handelt es sich um organische und anorganische Stoffe, die von Straßenschmutz, Abgasen, Insekten, Lackkonservierungsstoffen und anderen Quellen herrühren können. Die hydrophobe Glasoberfläche bewirkt, daß der Wasserfilm hinter dem Wischgummi zu feinen Tröpfchen aufreißt, die aufgrund der Lichtstreuung zu einer starken Sichtbehinderung führen können. Diese hydrophoben dünnen Schichten sind außerordentlich haftfest und mit den bisher bekannten Verfahren während des Fuhrbetriebes nicht zu beseitigen. Diese bekannten Verfahren bestehen beispielsweise darin, daß fett- und schmutzlösende Substanzen dem Wischwasser zugegeben werden. Diese Zusätze können zwar durchaus zu einer gewissen Sichtverbesserung führen, jedoch handelt es sich bei diesem Effekt nur um eine relativ kurzzeitige Hydrophilierung durch Abdeckung der dünnen hydrophoben Schmutzschichten auf der Scheibenoberfläche. Eine Beseitigung dieser festhaftenden hydrophoben Substanzen wird nicht erreicht.

Ein gattungsgemäßes Verfahren zur Reinigung von Kraftfahrzeugfront- und -heckscheiben, die von einem Scheibenwischer überstrichen werden, wobei Wasser und ein Reinigungsmittel auf die Glasscheibe aufgebracht werden, ist aus GB-A-1 366 910 bekannt. Nach dem Auftrag des Glasreinigungsmittels wird dabei der die auf den Scheiben befindlichen Schmutzschichten entfernende Reinigungsvorgang durch den Scheibenwischer ausgeführt.

Weiterhin ist aus GB-A-2 224 743 bekannt, ein Poliermittel als Entfettungsmittel in Form einer Paste oder Emulsion einzusetzen, die auch einen Bestandteil zum Polieren der Glasoberflächen enthält. Dieses Mittel wird in der für Poliervorgänge üblichen Weise von Zeit zu Zeit mit Hilfe eines Tuches manuell auf die Glasscheibe aufgebracht. Nach dem Poliervorgang erfolgt dann ein Trocknen der Glasscheibe, bevor verbliebene pulverförmige Rückstände von dem eingesetzten Mittel manuell mit einem trockenen Tuch entfernt werden.

Aus GB-A-2 004 458 ist daneben ein Verfahren zur Reinigung von Kraftfahrzeugscheinwerferscheiben bekannt, bei dem zur Entfernung von angelagertem Schmutz ein Reinigungsmittel auf die Kraftfahrzeugscheinwerferscheibe aufgebracht wird.

Die Reinigung erfolgt hier einerseits durch die aufgebrachte Reinigungsflüssigkeit und andererseits durch zusätzliche Schrubbelemente zum mechanischen Entfernen von angebackenem Schmutz, wobei die Schrubbelemente Bestandteil des Scheibenwischers sind.

Aus der US-A 4 045 838 ist ein verbessertes Scheibenwischblatt bekannt, dessen Lippenoberfläche zur Erzielung eines niedrigen Reibungskoeffizienten mit einer Beschichtung eines elastomeren plastischen Materials mit niedrigem Reibungskoeffizienten versehen ist. Durch diese Oberflächenbeschichtung soll erreicht werden, daß gleichzeitig der Reibungswiderstand verringert und dennoch der Reinigungseffekt des Wischblattes verbessert wird.

Weiterhin ist aus der DD-C 241 083, der DD-C 275 878 und der JP-A 212 474/88 jeweils ein Verfahren zum Polieren von Oberflächen bekannt. Diese bekannten Verfahren werden benutzt zur Reinigung von Glasoberflächen wie beispielsweise Kristalloberflächen oder Oberflächen von optischen Bauteilen und ebenso zum Polieren von Keramikoberflächen, wobei durch die Zusammensetzung des Poliermittels und die Art des Poliervorgangs gleichzeitig ein hoher Glasabtrag und eine hohe Poliergenauigkeit angestrebt werden. Die Möglichkeit der Anwendung eines derartigen Verfahrens zur Reinigung von Kraftfahrzeugscheiben ist den Druckschriften nicht zu entnehmen und grundsätzlich auch nicht naheliegend.

### Vorteile der Erfindung

Das erfindungsgemäße Verfahren mit den kennzeichnenden Merkmalen des Hauptanspruchs hat den Vorteil, daß hydrophobe Schmutzschichten während des Wischvorganges durch einen Poliervorgang abgetragen werden, so daß die Glasoberfläche wieder hydrophil wird und das Aufreißen des Wasserfilms zu feinen Tröpfchen verhindert werden kann. Hierdurch kann eine merkliche Sichtverbesserung auch für längere Dauer erreicht werden, wobei diese Sichtverbesserung bei einem Kraftfahrzeug während des Fahrbetriebs erreicht werden kann. Da die hydrophoben Schichten entfernt und nicht nur kurzzeitig überdeckt werden, kann eine langanhaltende Wirkung erreicht werden.

Durch die in den Unteransprüchen aufgeführten Maßnahmen sind vorteilhafte Weiterbildungen und Verbesserungen des im Hauptanspruch angegebenen Verfahrens möglich.

Das eingesetzte Glaspoliermittel kann dem Wischwasser zugesetzt werden, wodurch nur geringe Veränderungen bei einer herkömmlichen Wischanlage erforderlich sind. Es ist jedoch auch möglich, das Glaspoliermittel aus einem separaten Vorratsbehälter auf die Glasscheibe zu spritzen. Separate Vorratsbehälter für Reinigungsmittel sind ebenfalls bereits in verschiedenen Fahrzeugen serienmäßig vorgesehen, so daß auch hier die vorhandene Wischanlage eingesetzt werden kann.

Ein manueller Auftrag kann auch direkt auf der Glasscheibe erfolgen.

### Zeichnung

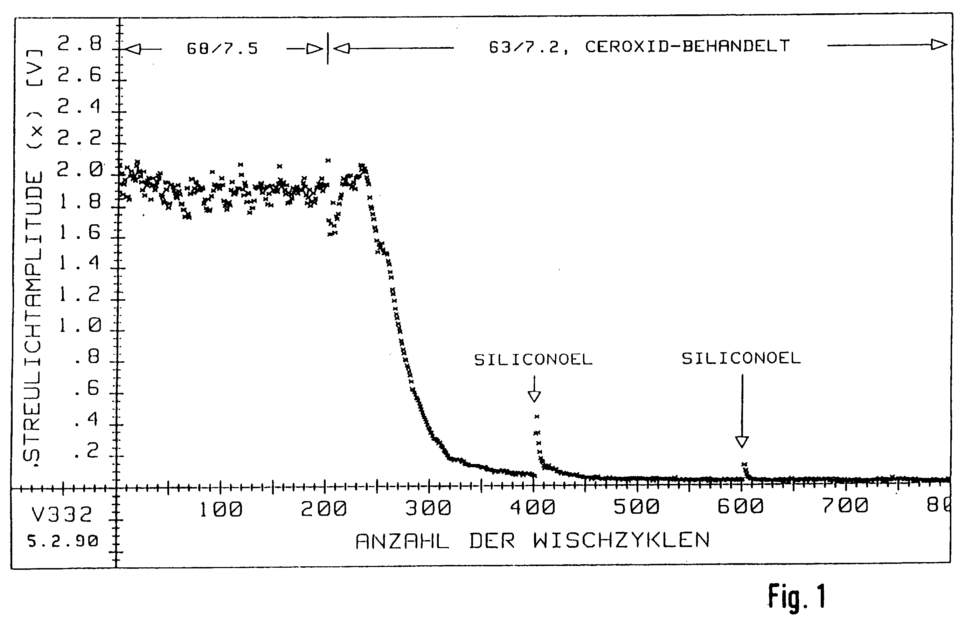

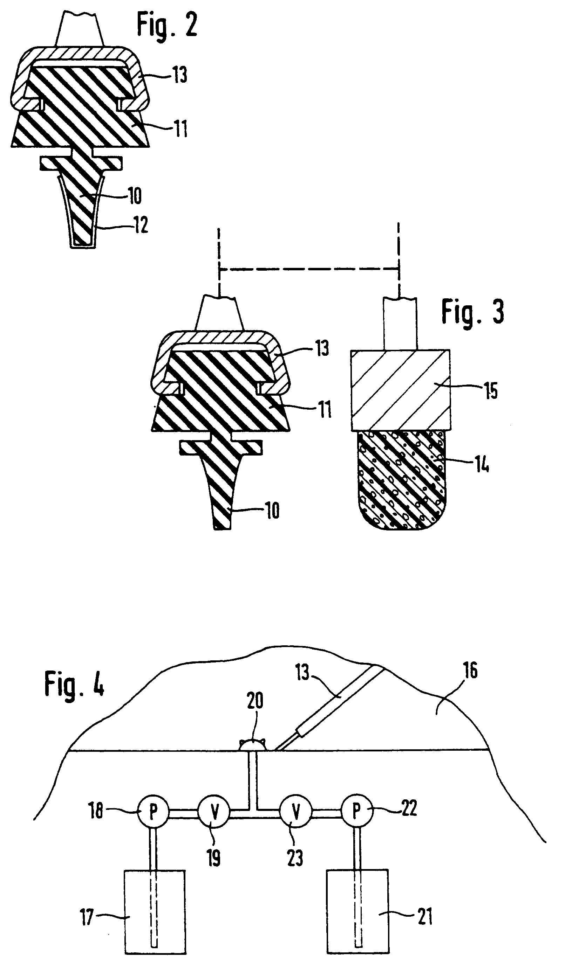

Ausführungsbeispiele der Erfindung sind in den Figuren 1 und 4 in der Zeichnung dargestellt und in der nachfolgenden Beschreibung näher erläutert. Es zeigen:
- Figur 1: ein Versuchsdiagramm zur Erläuterung der Wirkungsweise,
- Figur 2: eine Querschnittsdarstellung einer beschichteten Gummiwischlippe,
- Figur 3: eine herkömmliche Wischlippe und eine separate Polierlippe im Querschnitt und
- Figur 4: eine schematische Darstellung einer Wischanlage mit separaten Vorratsbehältern.

Beiden Figuren 2 und 3 handelt es sich nicht um Ausführungsarten der Erfindung.

Die der Erfindung zugrundeliegende Idee beruht darauf, die auf einer Glasscheibe festhaftenden hydrophoben Substanzen durch ein für Glasoberflächen geeignetes Poliermittel zu entfernen, um eine hydrophile Scheibenoberfläche zu schaffen, bei der der hinter einem Wischgummi eines Scheibenwischers zurückbleibende dünne Wasserfilm nicht reißt. Hierdurch entstehen gute, schleierfreie Sichtverhältnisse. Als Poliermittel kommen alle für die Glaspolitur geeigneten Stoffe in Frage, wobei in der Hauptsache die zu diesem Zweck gebräuchlichen Metalloxide wie Fe₃O₄, Fe₂O₃, Al₂O₃, Cr₂O₃, CeO₂, ZrO₂, TiO₂, SnO₂, SiO₂ geeignet sind. Von diesen ist nach praktischen Erfahrungen und theoretischen Überlegungen Ceroxid (CeO₂) die wirksamste Substanz.

Der Poliervorgang an einer Glasoberfläche ist ein komplizierter Vorgang. Man geht davon aus, daß ein Zusammenwirken von mechanischer Abtragung, plastischer Verformung der Oberflächenschicht und chemischen Vorgängen vorliegt. Bei der tribochemischen Reibungs-Verschleiß-Poliertheorie wird davon ausgegangen, daß eine örtliche "Verschweißung" der Polierkörner mit der Glasoberfläche erfolgt, und daß durch Abreißen an den Verbindungsstellen ein molekularer Abtrag erfolgt. Beim Poliervorgang treten vier Beteiligte in Erscheinung: die Glasoberfläche, das Poliermittel, die Suspensionsflüssigkeit und der Poliermittelträger. Aus diesem Grunde kann die Reinigungswirkung bei geringst möglichem Glasverschleiß unter Berücksichtigung unter anderem der Reibgeschwindigkeit, des Anpreßdrucks, der Härte des Poliermittelträgers und der mittleren Teilchengröße des Poliermittels optimiert werden. Für die praktische Anwendung hat sich eine Teilchengröße im Bereich zwischen 10nm und 1µm als besonders günstig erwiesen.

Experimente haben die Reinigungswirkung von Glaspoliermitteln an hydrophoben Glasoberflächen eindeutig belegt. Hierbei wurden bei den einzelnen Wischzyklen (Aufspritzen von Wasser, Wischbewegung eines Scheibenwischers) die Streulichtintensität als Maß für den auf der Scheibe gebildeten Tröpfchenschleier und damit als indirektes Maß für die Scheibenhydrophobie registriert. Als Glaspoliermittel wurde dabei Ceroxid verwendet. Figur 1 zeigt den Verlauf der Streulichtamplitude nach einer bestimmten Anzahl von Wischzyklen auf eine hydrophoben Scheibe. Die Hydrophobierung wurde auf einer Floatglasscheibe durch Auftragung von Silikonöl und anschließendem Blankreiben mittels Papiertüchern erzeugt. Die Hydrophobie der Scheibenoberfläche offenbart sich direkt in den großen Kontaktwinkeln, die aufgelegte Wassertropfen mit der Scheibe bilden.

Während der ersten zweihundert Wischzyklen wurde unter Standardbedingungen (Wischgummihärte 68 Shore, Schneidmaß 7,5 mm, Auflagekraft (normiert) 16 N/m, Reibgeschwindigkeit 2 m/s, Anstellwinkel 0°) eine mittlere Streulichtamplitude von ca. 1,9 Volt registriert. Nach dem zweihundersten Wischzyklus wurde die Messung gestoppt und ein Wischgummi (63 Shore, Schneidmaß 7,2 mm) eingebaut, dessen Lippe auf einer Glasscheibe mit in Wasser aufgeschlemmtem Ceroxid beschichtet worden war.

Während der nachfolgenden Wischbewegungen fiel die Streulichtamplitude recht schnell auf sehr kleine Werte. Die subjektive Beobachtung mit bloßem Auge bestätigte die Messung: der Schleier verschwindet vollständig, Wassertropfen spreiten auf der Glasoberfläche (Kontaktwinkel ungefähr 0°). Silikonöl, das jeweils nach dem vierhundertsten und sechshundertsten Wischzyklus aufgesprüht wurde, führte nur kurzzeitig zu einem Streulichtanstieg. Während weiterer achthundert Wischzyklen blieb die Scheibenoberfläche hydrophil. Bei diesem Meßvorgang muß berücksichtigt werden, daß das Aufsprühen von reinem Silikonöl eine extreme Verschmutzung darstellt, die in dieser gravierenden Form in der Praxis nicht zu erwarten ist.

Zum Aufbringen des Glaspoliermittels, insbesondere von Ceroxid gibt es verschiedene Alternativen. Gemäß Figur 2 ist eine Wischlippe 10 an einem Wischgummi 11 mit einer Schicht 12 aus Ceroxid versehen. Der Wischgummi 11 ist in üblicher Weise an einem Wischerarm 13 befestigt, der zur Vereinfachung nur teilweise dargestellt ist.

Die Schicht 12 aus Ceroxid oder einem anderem Metalloxid bzw. Glaspoliermittel kann durch Aufstreichen, Aufbürsten, Aufsprühen, durch ein Eintauchverfahren oder dergleichen auf die Wischlippe 10 aufgebracht werden. Dies kann während der Wischgummibearbeitung, z.B. bei der Graphitierung oder anstelle der Graphitierung erfolgen. Ceroxid-Pulver kann auch nachträglich in die Gummioberfläche mit oder ohne Temperatureinwirkung eingearbeitet werden, z.B. durch einen Walz- oder Preßvorgang. Eine weitere Möglichkeit besteht darin, nach der Formgebung, jedoch zweckmäßigerweise vor dem Schneiden, durch einen Spritzprozeß mit hoher mechanischer und/oder thermischer Energie Ceroxid in die Oberfläche einzuschießen, ähnlich einer Ionenimplantation. Selbstverständlich kann auch das Gummimaterial des Wischgummis 11 bereits bei der Herstellung mit Ceroxid bzw. einem Glaspoliermittel vermischt werden.

Gemäß Figur 3 ist eine separate Polierlippe 14 vorgesehen werden, die als Schaumstoffleiste ausgebildet ist. Auch andere Materialien kommen hierfür in Frage. Diese Schaumstoffleiste ist breiter ausgebildet, damit eine größere Menge Glaspoliermittel beim Wischvorgang aufgebracht werden kann, das im Schaumstoff oder dergleichen enthalten oder als Schicht aufgebracht ist. Die Polierlippe 14 ist an einer Halteleiste 15 befestigt, die Bestandteil des übrigen Scheibenwischers sein kann oder einen separaten Scheibenwischer bildet, der zur Intensivreinigung betätigt werden kann.

Ceroxid kann dem Wischwasser zugesetzt werden oder aus einem separaten Vorratsbehälter auf die Scheibe gespritzt werden. Dies ist in dem in Figur 4 dargestellten Ausführungsbeispiel schematisch dargestellt. Ein Wischerarm 13 überstreicht beim Wischvorgang eine Glasscheibe 16, z.B. die Front- oder Heckscheibe eines Kraftfahrzeuges. In einem ersten Behälter 17 enthaltenes Wischwasser wird mittels einer Pumpe 18 über ein Rückschlagventil 19 einer Spitzdüsenanordnung 20 zugeführt, von wo aus es auf die Glasscheibe 16 gespritzt wird. Weiterhin ist ein zweiter Behälter 21 zur Aufnahme einer Suspension von Ceroxid vorgesehen. Dieses wird über eine zweite Pumpe 22 und ein zweites Rückschlagventil 23 der Spritzdüsenanordnung 20 zugeführt. Durch wahlweise oder gleichzeitige Betätigung der Pumpen 18, 22 kann entweder nur Wischwasser oder zusätzlich die Ceroxid-Suspension zur Reinigung auf die Glasscheibe 16 aufgespritzt werden.

In bekannter Weise kann selbstverständlich auch eine einzige Pumpe und zwei Ventile in den beiden Leitungen zu den beiden Behältern 17 und 21 vorgesehen sein. Weiterhin können auch getrennte Spritz- oder Sprühdüsen vorgesehen sein, wobei der zweite Behälter 21 Ceroxid enthalten kann, das über eine entsprechend ausgebildete Düse auf die Scheibe aufgesprüht wird. Es ist auch möglich, über ein Ventil pulverförmiges Ceroxid dem Wischwasser in der Wischwasserleitung zuzuführen.

Wesentlich ist jeweils, daß Glaspoliermittel, insbesondere Ceroxid auf die zu behandelnde Glasscheibe aufgebracht wird, wobei der Scheibenwischer dann den Poliervorgang ausführt.

Eine weitere Möglichkeit der Realisierung besteht auch darin, Glaspoliermittel, insbesondere Ceroxid, in einer Flasche bereitzustellen. Das Glaspoliermittel wird dann direkt auf der Glasscheibe, manuell verteilt. Anschließend übernimmt dann der Scheibenwischer den Poliervorgang, wobei selbstverständlich auch ein zusätzlicher manueller Poliervorgang der Glasscheibe erfolgen kann. Zum Aufbringen des Glaspoliermittels können geeignete Hilfsmittel verwendet werden, wie ein Schwamm oder ein der Form der Wischlippe nachgebildetes Formteil zur besseren Aufbringung und Verteilung des Glaspoliermittels.

## Patentansprüche

1. Verfahren zur Reinigung von Kraftfahrzeugfront- und heckscheiben, die von einem Scheibenwischer überstrichen werden, wobei Wasser und ein Reinigungsmittel auf die Glasscheibe aufgebracht werden, dadurch gekennzeichnet, daß zur Hydrophilierung der Scheibenoberflache ein Glaspoliermittel in Form einer Suspension auf die Scheibe aufgebracht wird, wobei der hydrophobe Schmutzschichten abtragende Poliervorgang vom Scheibenwischer ausgeführt wird.

2. Verfahren nach Anspruch 1, dadurch gekennzeichnet, daß das Glaspoliermittel dem Wischwasser zugesetzt wird.

3. Verfahren nach Anspruch 1, dadurch gekennzeichnet, daß das Glaspoliermittel aus einem separaten Vorratsbehälter (21) auf die Glasscheibe (16) gespritzt wird.

4. Verfahren nach Anspruch 1, dadurch gekennzeichnet, daß das Glaspoliermittel von einem Behälter, wie einer Flasche auf der Glasscheibe (16) verteilt wird.

## Claims

1. Method for cleaning motor-vehicle windscreens and rear windows which are wiped by a wiper, with water and a cleaning agent being applied to the glass window, characterized in that a glass-polishing agent in the form of a suspension is applied to the window in order to make the window surface hydrophilic, the polishing process which removes hydrophobic dirt layers being carried out by the wiper.

2. Method according to Claim 1, characterized in that the glass-polishing agent is added to the wiper fluid.

3. Method according to Claim 1, characterized in that the glass-polishing agent is sprayed onto the glass window (16) from a separate storage container (21).

4. Method according to Claim 1, characterized in that the glass-polishing agent is distributed on the glass window (16) from a container, such as a bottle.

## Revendications

1. Procédé de nettoyage des pare-brise et des lunettes arrière de véhicules automobiles, balayés par un balai d'essuie-glace, en appliquant de l'eau et un agent de nettoyage sur la vitre,
caractérisé en ce que
pour hydrophiliser la surface de la vitre, on applique sur la vitre un agent de polissage du verre sous forme de suspension, et l'opération de polissage, qui enlève les couches de saletés hydrophobes, est assurée par le balai d'essuie-glace.

2. Procédé selon la revendication 1,
caractérisé en ce que
l'agent de polissage du verre est ajouté à l'eau de nettoyage.

3. Procédé selon la revendication 1,
caractérisé en ce que
l'agent de polissage du verre est projeté sur la vitre (16) à partir d'un réservoir (21) distinct.

4. Procédé selon la revendication 1,
caractérisé en ce que
l'agent de polissage du verre est distribué, à partir d'un réservoir tel qu'une bouteille, sur la vitre (16).
